# EUROPEAN PATENT SPECIFICATION

(11) **EP 1 968 593 B1**
(45) Date of publication and mention of the grant of the patent: **23.08.2017**
(21) Application number: 06829701.9
(22) Date of filing: 18.12.2006
(51) Int. Cl.: A61K 31/505, A61K 9/20, A61P 9/10, A61P 3/06, A61K 45/06

(54) **PHARMACEUTICAL COMPOSITION COMPRISING (E)-7-[4-(4-FLUOROPHENYL)-6-ISOPROPYL-2-[METHYL(METHYLSULFONYL)AMINO]PYRIMIDIN-5-YL-(3R,5S)-3,5-DIHYDROXYHEPT-6-ENOIC ACID**
PHARMAZEUTISCHE ZUSAMMENSETZUNG ENTHALTEND (E)-7-[4-(4-FLUOROPHENYL)-6-ISOPROPYL-2-[METHYL(METHYLSULFONYL)AMINO]PYRIMIDIN-5-YL-(3R,5S)-3,5-DIHYDROXYHEPT-6-ENSÄURE
COMPOSITION PHARMACEUTIQUE COMPRENANT DE L'ACIDE (E)-7-[4-(4-FLUOROPHENYL)-6-ISOPROPYL-2-[METHYL(METHYLSULFONYL)AMINO]PYRIMIDIN-5-YL-(3R,5S)-3,5-DIHYDROXYHEPT-6-ENOIQUE

(30) Priority: 20.12.2005 SI 200500344
(43) Date of publication of application: 17.09.2008
(73) Proprietor: LEK Pharmaceuticals d.d., 1526 Ljubljana (SI)
(72) Inventor: JAKLIC, Miha, Tomaz, 1262 Dol pri Ljubljani (SI); NAVERSNIK, Klemen, 1360 Vrhnika (SI)
(86) International application number: PCT/EP2006/012180
(87) International publication number: WO 2007/071357

(56) References cited:
- EP-A1- 0 521 471
- EP-A1- 1 185 274
- WO-A-02/089788
- WO-A-03/092729
- WO-A-2004/071403
- WO-A-2005/040134
- WO-A-2005/077916
- WO-A-2005/077917
- US-A1- 2005 187 234
- US-B1- 6 548 513
- "Core Data Sheet Crestor(TM) Tablets"[Online] 17 June 2003 (2003-06-17), XP002449400 Retrieved from the Internet: URL:http://www.crestor.info/gUserFiles/CRE STOR_CDS_10_40_mg_FINAL_170603.pdf> [retrieved on 2007-09-05]

## Description

### Field of the Invention

Present invention from the field of pharmaceutics relates to pharmaceutical composition comprising the hemicalcium salt of (E)-7-[4-(4-fluorophenyl)-6-isopropyl-2-[methyl(methylsulfonyl)amino]pyrimidin-5-yl]-(3R, 5S)-3,5-dihydroxyhept-6-enoic acid (hereinafter "the agent").

### Background of the Invention

The agent is a 3-hydroxy-3-methylglutaryl (HMG) CoA reductase inhibitor known from EP 521471 and formulated into a pharmaceutical composition can be used for (manufacturing the medicament for) treating hypercholesterolemia, hyperlipidproteinemia and atherosclerosis. A major issue associated with the bulk agent or formulated into a composition is that it is particularly sensitive to degradation. The major degradation products formed (as known from US 6548513) are the lactone (N-{4-(4-Fluoro-phenyl)-5-[2-(4-hydroxy-6-oxo-tetrtahydropyran-2-yl)-vinyl]-6-isopropyl-pyrimidin-2-yl}-N-methyl-methanesulfonamide) and the oxidation product (7-[4-(4-fluorophenyl)-6-isopropyl-2-[methyl(methylsulfonyl)amino]pyrimidin-5-yl]-3-hydroxy-5-oxo-hept-6-enoic acid). Also, when exposed to light, the agent undergoes degradation to two diastereomeric cyclic products, described in US 2005/0187234 A1. The mentioned degradations of the agent under conditions of humidity, acidity, oxygen and light is a challenge for the manufacture of a pharmaceutical formulation, stable enough for ordinary storage conditions.

This stabilization of the agent or chemically similar compounds, especially those belonging to HMG-CoA reductase inhibitors, could be achieved by controlling pH in a formulation (by addition of components such as a carbonate or bicarbonate) and by adding to composition a stabilizing inorganic salt, particularly tribasic tribasic calcium phosphate. Antioxidants, such as butylated hydroxytoluene may also be used to hinder oxidation of the agent. Another option is to stabilize a pharmaceutical composition using an amino sugar. Pharmaceutical composition of the agent currently marketed under name Crestor (tm) contains 5, 10, 20, or 40 mg of the agent, tribasic calcium phosphate as the stabilizing inorganic salt and the following inactive ingredients: microcrystalline cellulose, lactose monohydrate, crospovidone,

magnesium stearate, hypromellose, triacetin, titanium dioxide, yellow ferric oxide, and red ferric oxide.

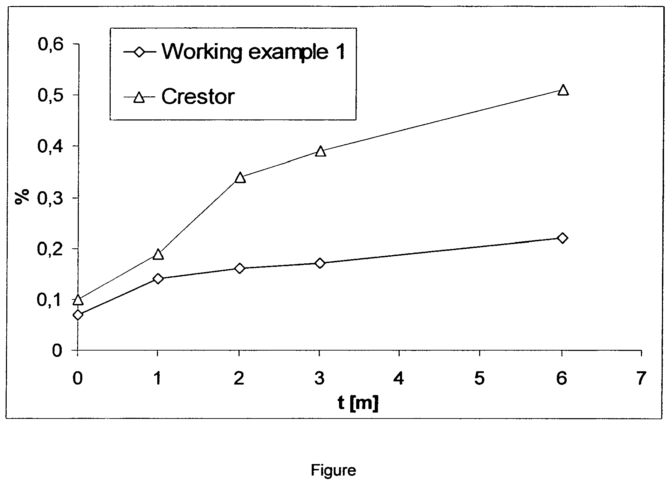

### Description of the figure

Figure represents the comparison of the amount of the main degradation product (lactone form of the agent, as measured by HPLC), which is formed if the compositions corresponding to currently marketed formulation and the composition in accordance with our invention are subjected to accelerated stability program (as proposed by ICH guidelines: 40°C and 75 % relative humidity, stored in the primary package). The y axis represents the % of the formed degradation product (lactone), and x axis the time In months.

### Disclosure of the Invention

In one aspect the invention provides pharmaceutical composition comprising a hemicalcium salt of (E)-7-14-(4-fluorophenyl)-6-isopropyl-2-[methyl(methylsulfonyl)amino]pyrimidin-5-yl]-(3R, 5S)-3,5-dihydroxyhept-6-enoic acid, wherein
said hemicalcium salt is present in amount of 5-20% wt;
lactose Is present in an amount of 40 - 60% wt;
silicified microcrystalline cellulose is present In an amount of 20-30% wt;
corn starch Is present in amount of 1 to 25%,
at least one glidant, selected from talc and colloidal silicium dioxide, is present in an amount of 0.5 - 5% wt,
at least one lubricant, selected from sodium stearyl fumarate and glyceryl behenate is present in an amount of 0.1 - 3% wt, and
optionally sodium starch glycolate is present in an amount of 0 - 5% wt.

A pharmaceutical composition of the present Invention comprising (E)-7-[4-(4-fluorophenyl)-B-isopropyl-2-[methyl(methyisulfonyl)amino]pyrimidin-5-yl]-(3R, 5S)-3,5-dihydroxyhept-6-enoic acid or a pharmaceutically-acceptable salt thereof contains less than 0,05% as measured by HPLC of the 7-[4-(4-fluorophenyl)-6-isopropyl-2-[methyl(methylsulfonyl)amino]pyrimidin-5-yl]-3-hydroxy-5-oxo-hept-6-enoic acid.

A pharmaceutical composition of the present invention comprising the agent contains less than 0,5% as measured by HPLC of the N-{4-(4-Fluoro-phenyl)-5-[2-(4-hydroxy-6-oxo-tetrahydropyran-2-yl)-vinyl]-6-isopropyl-pyrimidin-2-yl}-N-methyl-methanesulfonamide and also less than 0,05% as measured by HPLC of the 7-[4-(4-fluorophenyl)-6-isopropyl-2-[methyl(methylsulfonyl)amino]pyrimidin-5-yl]3-hydroxy-5-axo-hept-6-enoic acid.

The composition may be film coated wherein said coating comprises HPMC, HPC, polyethylene glycol and talc.

Additionally the Invention Is a process for preparing a pharmaceutical composition comprising the hemicalcium salt of (E)-7-[4-(4-fluorophenyl)-6-isopropyl-6-isopropyl-2-methyl(methylsulfonyl)amino]pyrimidin-5-yl]-(3R, 5S)-3,5-dihydroxyhept-6-enoic acid characterized In that the process comprises following steps: a) dry blending said hemicalcium salt and excipients mixture, wherein this mixture comprises lactose, silicified microcrystalline cellulose and corn starch; b) (optionally) mixing therein additional excipients; c) mixing therein a lubricant selected from sodium stearyl fumarate or glyceryl behenate specifically glyceryl behenate; d) compressing obtained powder mixture Into tablets; e) (optionally) coating of tablets prepared In preceding steps, wherein the amounts by weight to the weight of the final compositions are: hemicalcium salt of (E)-7-[4-(4-fluorophenyl)-6-isopropyl-2-[methyl(methylsulfonyl)amino]pyrimidin-5-yl]-(*3R,5S*)-3,5-dihydroxyhept-6-enoic acid: 5-20% wt; lactose: 40 - 60% wt; silicified microcrystalline cellulose: 20-30% wt; and corn starch: 1 to 25 % wt relative to the weight of the composition and (optionally) film coating. The invention is embodied in the use of the pharmaceutical composition as claimed for treating hypercholesterolemia, hyperlipidproteinemia and atherosclerosis. The invention is embodied in a novel pharmaceutical composition having the following advantages: the composition inhibits in long term (i.e. during the shelf life of a medicinal product) the formation of the agent's degradation products; the formulation is not alkaline in nature (measured by the pH of an aqueous dispersion of the formulation, which is 6.2) neither are any basifying agents added to hinder the degradation of the agent; the pharmaceutical composition allows a technically feasible process of creating a formulation with suitable biopharmaceutical properties.

Thus the lack of alkaline ingredients on one hand minimizes the potential of impaired in-vivo absorption of the agent due to the changes of the gastrointestinal pH and on the other side such composition is advantageous for patient because an alkaline composition would have adverse effects on gastric mucosa. The pH of the aqueous solution or dispersion of the composition In accordance with our Invention will be substantially neutral, preferably between 6 and 8, as determined if a tablet containing 40 mg of agent is dispersed in 40 ml of water and measured by glass electrode pH meter. Compositions can be manufactured by established technological procedures, preferably direct compression or wet granulation followed by tabletting and film coating for manufacturing of finished dosage form (e.g. tablet) and at the same time demonstrates suitable biopharmaceutical properties such as comparable dissolution and/or bioequivalence to Crestor.

The present Invention combines in a pharmaceutical composition the agent with the ingredients that stabilize the agent. Ingredients are selected according to two stabilizing properties. In the first group are the ingredients which were found to inhibit oxidation of the agent: corn starch, silicified microcrystalline cellulose, croscarmellose sodium, and hypromellose. In the second group are the ingredients which were found to inhibit the formation of the lactone form of the agent: corn starch, mannitol, hydroxypropyl cellulose and hypromellose. The formation of the degradation products of the agent under the influence of light can also be additionally hindered using pharmaceutically acceptable pigments or colorants, for instance In a tablet coating.

Preferably selection of Ingredients from both groups and a protection from light results in a pharmaceutical composition wherein the agent is stabilized. In such formulation, the agent stays stable with respect to oxidation, formation of the lactone and formation of degradation products, preferably over a period of years, more preferably months.

A pharmaceutical composition of the present invention comprises the agent, one or more ingredients from the first group (oxidation inhibiting ingredients), one or more ingredients from the second group (lactonization Inhibiting ingredients) and one or more fillers (also known as diluents), binders, disintegrants or lubricants. Additional, conventional excipients may be added: for example preservatives, silica flow conditioners, antiadherents and stabilizers. It will be appreciated that a particular excipient may act different roles in a pharmaceutical composition, e.g. as a filer, a binder and a disintegrant.

The above stabilizing substances can also have a function of a filer (diluents), binder, or disintegrant. In addition to lactose one or more additional fillers may be present. Suitable additional fillers include, for example, cellulose and its derivatives (e.g. microcrystalline cellulose, powdered cellulose), modified starches, polyols, inorganic salts, or any other fillers commonly used in the art. Suitable binders include, for example, polyvinylpyrrolidone, gum acacia, gum tragacanth, guar gum, pectin, microcrystalline cellulose, methylcellulose, carboxymethylcellulose, hydroxypropyl methylcellulose, hydroxyethyl cellulose, hydroxypropyl cellulose, gelatin and sodium alginate. Suitable disintegrants include, for example, crosscarmellose sodium, crospovidone, sodium starch glycollate, hydroxypropyl methylcellulose and hydroxypropyl cellulose. Suitable lubricants include, for example, magnesium stearate, stearic acid, palmitic acid, calcium stearate, talc, carnauba wax, hydrogenated vegetable oils, mineral oil, glyceryl behanate, polyethylene glycols and sodium stearyl fumarate.

The pharmaceutical composition of the invention may be prepared using standard techniques and manufacturing processes generally known In the art, for example by dry blending the components. The components of the blend prior to blending, or the blend itself, may be passed through a mesh screen. Conveniently a lubricant, may also be added to the blend and blending continued until a homogeneous mixture is obtained. The mixture is then compressed into tablets. Alternatively, a wet granulation technique can be employed.

Pharmaceutical compositions comprising (E)-7-[4-(4-fluorophenyl)-6-isopropyl-2-[methyl(methylsulfonyl)amino]pyrimidin-5-yl]-(3R, 5S)-3,5-dihydroxyhept-6-enoic acid hemicalcium salt and Ingredients from aforesaid first group and second group are prepared by dry blending the agent and all excipients (inactive ingredients) except lubricant in a double cone mixer. Next a lubricant, in one embodiment glyceryl behenate, and in another embodiment sodium stearyl fumarate is added to the mixture and blended for a short period of time, such as needed to substantially homogenize the mixture, e.g. up to 5 miutes. The mixture is then compressed Into tablets and film coated with a conventional coating composition consisting of film forming polymer such as hypromellose or hydroxypropyl cellulose, film softener such as polyethylene glycol, pigments, talc.

A typical composition in accordance with our invention will comprise (wt.)

| | |
|---|---|
| agent (hemicalcium salt) | 5 - 20 % |
| lactose | 40 - 60 % |
| silicified microcrystalline cellulose | 20 - 30 % |
| corn starch | 1 - 25 % |

and further:

| | |
|---|---|
| a glidant (talc and/or colloidal silicium dioxide) | 0,5 - 5 % |
| a lubricant (sodium stearyl fumarate and/or glyceryl behenate) | 0,1 - 3 % |

and optionally further

| | |
|---|---|
| sodium starch glycolate | 0 - 5 % |

A tablet coating may then be applied, for example by spray-coating, with a water-ethanol based film coating formulation. Coating ingredient combinations are readily available. In an embodiment of the invention coatings containing pigments or colorants reduce the rate of formation of photo degradation products of the agent.

### Experimental part

The bulk agent is subjected to stress conditions, such as elevated temperature (40°C and above), elevated humidity (75 % or higher relative humidity, open dish conditions), oxygen atmosphere or solutions with different pH. An HPLC analysis, capable of resolving the agent and its degradation products is then employed, to quantify the amount (given as a mass percentage relative to the agent) of degradation products in the stressed samples. Good chromatographic resolution can be achieved on a C18 reversed phase column with acidic phosphate buffer and an increasing gradient of acetonitrile and tetrahydrofuran. Degradation products are quantified using UV detection at 242 nm. The reporting limit of the degradation products has been set at 0,05 %. The % reported for HPLC analysis are in general area %.

When bulk (E)-7-[4-(4-fluorophenyl)-6-isopropyl-2-[methyl(methylsulfonyl)amino]pyrimidin-5-yl]-(3R, 5S)-3,5-dihydroxyhept-6-enoic acid hemicalcium salt is exposed to stress conditions the compound chemically degrades, showing its instability as demonstrated by following findings:

| stress condition | amount of lactone (%) | amount of oxidation product (%) |
|---|---|---|
| aqueous solution of the agent, buffered to pH = 5 at room temperature (24 hours) | 2,30 | <0,05 |
| the agent under oxygen at 60°C for 14 days | 0,17 | 0,71 |

The results of exposing to the same stress condition a mixture of agent and commonly used excipients such as lactose, crosslinked carboxymethylcellulose sodium or crosslinked PVP are substantially similar to results of the bulk agent subjected to the stress conditions.

However exposing mixtures of agent and the ingredients from the aforementioned first and second group, the amounts of lactone formed are substantially lower then as with the agent alone, showing their stabilizing property.

When a binary mixture (w 1:1) of the agent and any of the ingredients from the first group is stored under oxygen at 60°C for 14 days, no oxidation product is formed compared to 0,71% formed on exposing the bulk agent.

Two working examples of stable formulations are presented, followed by two reference examples (first with commonly used tableting excipients and second including an alkalizing excipient).

### WORKING EXAMPLE 1

The following pharmaceutical composition is a novel composition, prepared by the process as described above, using ingredients from both two groups of stabilizing ingredients.

| **Ingredient** | **Function** | **w %** |
|---|---|---|
| The Agent (calcium salt) | active | 9,2 |
| Lactose | filler | 55,9 |
| Silicified Microcrystalline Cellulose | filler, binder, disintegrant, active stabilizer | 24,4 |
| Corn Starch | filler, binder, disintegrant, active stabilizer | 3,6 |
| Sodium Starch Glycolate | disintegrant, active stabilizer | 2,4 |
| Talc | glidant | 2,7 |
| Glyceryl Behenate | lubricant | 1,8 |

The pH of the formulation is 6,2. The amount of lactone is only 0.50% after 14 days at 40°C and 75% relative humidity (open dish). The oxidation product is not formed.

The amount of lactone does not increase above 0,25% after 6 months at 40°C in impermeable package. The amount of the oxidation product after 6 months at 40°C in impermeable package is only 0,05% or below. Comparatively the amounts of lactone and oxidation product in a pharmaceutical composition of the agent such as the one currently marketed (Figure) are 0,51 % and 0,38 %, respectively.

The tablet film coating (2,5% coating on weight of coated tablet) consisting of hypromellose, hydroxypropyl cellulose, polyethylene glycol, pigments and talc had no substantial effect on formation of lactone or oxidation product.

### WORKING EXAMPLE 2

| **Ingredient** | **Function** | **w %** |
|---|---|---|
| The Agent (calcium salt) | active | 9,2 |
| Lactose | filler | 48,9 |
| Colloidal Silicum Dioxide | glidant | 0,3 |
| Silicified Microcrystalline Cellulose | filler, binder, disintegrant, active stabilizer | 25,0 |
| Corn Starch | filler, binder, disintegrant, active stabilizer | 15,0 |
| Talc | glidant | 1,0 |
| Sodium Stearyl Fumarate | lubricant | 0,6 |

Tablets are film coated (2,5% coating on weight of coated tablet) consisting of hypromellose, hydroxypropyl cellulose, polyethylene glycol, pigments and talc.

The pH of the formulation is 6,3. The amount of lactone is only 0.35 % - 0.43 % after 14 days at 40°C and 75% relative humidity (open dish). The oxidation product is not formed.

### REFERENCE EXAMPLE 1

The following is a composition comprising agent and commonly used excipients as suggested by a lactose producer - DMV. Tablets are prepared according to same process as in above working examples. Amount of degradation products indicate instability of the agent in a composition.

| **Ingredient** | **Function** | **w %** |
|---|---|---|
| The Agent (calcium salt) | active | 9,4 |
| Lactose Anhydrous | filler, binder | 41,7 |
| Colloidal Silicium Dioxide | glidant | 0,3 |
| Lactose, Sieved | filler | 41,7 |
| Croscarmellose Sodium | disintegrant | 4,0 |
| Talc | glidant | 1,0 |
| Glyceryl Behenate | lubricant | 1,8 |

The amount of lactone is 2.32% after 14 days at 40°C and 75% relative humidity (open dish).

### REFERENCE EXAMPLE 2

The following is a composition comprising agent and commonly used excipients as taught by a textbooks in this category: Pharmaceutical dosage forms: Tablets vol. 1 (Herbert, Lieberman, Lachman, and Schwartz; Marcel Dekker, New York and Basel; second edition, 1989. To this composition an alkalizing agent was added. Tablets are prepared according to same process as in above working examples.

| **Ingredient** | **Function** | **w %** |
|---|---|---|
| The Agent (calcium salt) | active | 12,8 |
| Sodium carbonate decahydrate | alkalizing agent | 1,8 |
| Lactose Anhydrous | filler, binder | 46,1 |
| Colloidal Silicium Dioxide | glidant | 3,7 |
| Microcrystalline Cellulose | filler, binder, disintegrant | 30,9 |
| Croslinked Povidone | disintegrant | 2,8 |
| Glyceryl behenate | lubricant | 1,9 |

The composition shows the agent is stable in presence of alkalizing agent. The amount of lactone is 0.07 % after 1 month at 40°C and 75% relative humidity, open dish. The good stability of the sample is believed due to its alkalinity (pH = 9,9)

### REFERENCE EXAMPLE 3

In parallel the stability of the currently marketed product has been assessed. The amount of lactone has increased from 0.10 % to is 0.35 % after 1 month at 40°C and 75% relative humidity, and from 0.10 % to is 0.40 % after 10 days at 60°C exposed to oxygen. The amount of oxidation product remain the same 0.3% under first conditions and increased to 0,4% under second.

## Claims

1. A pharmaceutical composition comprising a hemicalcium salt of (E)-7-[4-(4-fluorophenyl)-6-isopropyl-2-[methyl(methylsulfonyl)amino]pyrimidin-5-yl]-(3R, 5S)-3,5-dihydroxyhept-6-enoic acid, wherein
said hemicalcium salt is present In amount of 5-20% wt;
lactose is present In an amount of 40 - 60% wt;
silicifed microcrystalline cellulose is present in an amount of 20-30% wt;
corn starch is present in an amount of 1 to 25%,
at least one glidant, selected from talc and colloidal silicium dioxide, is present In an amount of 0.5-5% wt,
at least one lubricant, selected from sodium stearyl fumarate and glyceryl behenate is present in an amount of 0.1 - 3% wt, and
optionally sodium starch glycolate is present In an amount of 0 - 5% wt.

2. The pharmaceutical composition according to claim 1, which is coated by a film coating.

3. The pharmaceutical composition according to the previous claim, wherein said coating comprises HPMC, HPC, polyethylene glycol and talc.

4. A process for preparing a pharmaceutical composition comprising the hemicalcium salt of (E)-7-[4-(4-fluorophenyl)-6-isopropyl-2-[methyl(methylsulfonyl)amino]pyrimidin-5-yl]-(3R, 5S)-3,5-dihydroxyhept-6-enoic acid **characterized in that** the process comprises following steps:
a) dry blending the hemicalcium salt of (E)-7-[4-(4-fluorophenyl)-6-isopropyl-2-Emethyl(methylsulfonyl)amino]pyrimidin-5-yl]-(3R, 5S)-3,5-dihydroxyhept-6-enoic acid and excipients mixture, wherein this mixture comprises lactose, silicified microcrystalline cellulose and corn starch;
b) (optionally) mixing therein additional excipients;
c) mixing therein a lubricant selected from sodium stearil fumarate or glyceryl behenate
d) compressing obtained powder mixture into tablets;
e) (optionally) coating of tablets prepared In preceding steps,
wherein the amounts by weight to the weight of the final compositions are:
hemicalcium salt of (E)-7-14-(4-fluorophenyl)-6-isopropyl-2-[methyl(methylsulfonyl)amino]pyrimidin-5-yl]-(3R, 5S)-3,5-dihydroxyhept-6-enoic acid: 5-20%; lactose: 40 - 60%;
silicified microcrystalline cellulose: 20-30%; and
corn starch: 1 to 25%.

5. The process according to claim 4, wherein the lubricant is glyceryl behenate

6. The pharmaceutical composition as defined in any of claims 1 to 3 for use In the treatment of hypercholesterolemia, hyperlipidproteinemia and atherosclerosis.

## Patentansprüche

1. Pharmazeutische Zusammensetzung umfassend ein Hemicalzium-Salz von (E)-7-[4-(4-Fluorphenyl)-6-isopropyl-2-[methyl(methylsulfonyl)amino]pyrimidin-5-yl]-(3R,5S)-3,5-dihydroxyhept-6-ensäure, wobei das Hemicalzium-Salz in einer Menge von 5-20 Gew.-% vorhanden ist;
Laktose in einer Menge von 40-60 Gew.-% vorhanden ist; Silicifizierte mikrokristalline Cellulose in einer Menge von 20-30 Gew.-% vorhanden ist;
Maisstärke in einer Menge von 1-25% vorhanden ist, mindestens ein Gleitmittel, ausgewählt aus Talk und kolloidalem Siliziumdioxid, in einer Menge von 0,5-5 Gew.-% vorhanden ist,
mindestens ein Schmiermittel, ausgewählt aus Natriumstearylfumarat und Glycerylbehenat in einer Menge von 0,1-3 Gew.-% vorhanden ist, und
gegebenenfalls Natriumstärkeglykolat in einer Menge von 0-5 Ges.-% vorhanden ist.

2. Pharmazeutische Zusammensetzung gemäß Anspruch 1, welche durch eine Filmbeschichtung beschichtet ist.

3. Pharmazeutische Zusammensetzung gemäß dem vorhergehenden Anspruch, wobei die Beschichtung HPMC, HPC, Polyethylenglykol und Talk umfasst.

4. Verfahren zur Herstellung einer pharmazeutischen Zusammensetzung umfassend das Hemicalzium-Salz von (E)-7-[4-(4-Fluorphenyl)-6-isopropyl-2-[methyl(methylsulfonyl)amino]pyrimidin-5-yl]-(3R,5S)-3,5-dihydroxyhept-6-ensäure, **dadurch gekennzeichnet, dass** das Verfahren folgende Schritte umfasst:
a) Trockenmischen des Hemicalzium-Salzes von (E)-7-[4-(4-Fluorphenyl)-6-isopropyl-2-[methyl(methylsulfonyl)amino]pyrimidin-5-yl]-(3R,5S)-3,5-dihydroxyhept-6-ensäure und einer Hilfsstoffe-Mischung, wobei diese Mischung Laktose, silicifizierte mikrokristalline cellulose und Maisstärke umfasst;
b) (gegebenenfalls) Einmischen zusätzlicher Hilfsstoffe;
c) Einmischen eines Schmiermittels ausgewählt aus Natriumstearylfumarat oder Glycerylbehenat
d) Verpressen der erhaltenen Pulvermischungen zu Tabletten;
e) (gegebenenfalls) Beschichten der in den vorhergehenden Schritten hergestellten Tabletten,
wobei die Gewichtsmengen bezogen auf das Gewicht der finalen Zusammensetzungen sind:
Hemicalzium-Salz von (E)-7-[4-(4-Fluorphenyl)-6-isopropyl-2-[methyl(methylsulfonyl)amino]pyrimidin-5-yl]-(3R,5S)-3,5-dihydroxyhept-6-ensäure: 5-20%;
Laktose: 40-60%;
Silicifizierte mikrokristalline Cellulose: 20-30%; und
Maisstärke: 1 bis 25%.

5. Verfahren gemäß Anspruch 4, wobei das Schmiermittel Glycerylbehenat ist.

6. Pharmazeutische Zusammensetzung die in irgendeinem der Ansprüche 1 bis 3 definiert zur Verwendung bei der Behandlung von Hypercholesterolämie, Hyperlipidproteinämie und Atherosklerose.

## Revendications

1. Composition pharmaceutique comprenant un sel d'hémicalcium de l'acide (E)-7-[4-(4-fluorophényl)-6-isopropyl-2-[méthyl(méthylsulfonyl)amino]pyrimidin-5-yl]-(3R, 5S)-3,5-dihydroxyhept-6-énoïque, dans lequel
ledit sel d'hémicalcium est présent en une quantité de 5 à 20% en poids ;
du lactose est présent en une quantité de 40 à 60% en poids ;
de la cellulose microcristalline silicifiée est présente en une quantité de 20 à 30 % en poids ;
de l'amidon de maïs est présent en une quantité de 1 à 25 %,
au moins un agent de glissement, sélectionné parmi le talc et le dioxyde de silicium colloïdal, est présent en une quantité de 0,5 à 5 % en poids,
au moins un lubrifiant, sélectionné parmi le fumarate de stéaryle de sodium et le béhénate de glycéryle est présent en une quantité de 0,1 à 3 % en poids, et éventuellement du glycolate d'amidon de sodium est présent en une quantité de 0 à 5 % en poids.

2. Composition pharmaceutique selon la revendication 1, qui est revêtue par un revêtement en film.

3. Composition pharmaceutique selon la revendication précédente, dans laquelle ledit revêtement comprend de l'HPMC, de l'HPC, du polyéthylène glycol et du talc.

4. Procédé de préparation d'une composition pharmaceutique comprenant le sel d'hémicalcium de l'acide (E)-7-[4-(4-fluorophényl)-6-isopropyl-2-[méthyl(méthylsulfonyl)amino]pyrimidin-5-yl]-(3R, 5S)-3,5-dihydroxyhept-6-énoïque **caractérisé en ce que** le procédé comprend les étapes suivantes :
a) mélanger à sec le sel d'hémicalcium de l'acide (E)-7-[4-(4-fluorophényl)-6-isoprapyl-2-[méthyl(méthylsulfonyl)amino]pyrimidin-5-yl]-(3R, 5S)-3,5-dihydroxyhept-6-énoïque et un mélange d'excipients, dans lequel ce mélange comprend du lactose, de la cellulose microcristalline silicifiée et de l'amidon de maïs ;
b) (éventuellement) mélanger avec ceux-ci des excipients supplémentaires;
c) mélanger avec ceux-ci un lubrifiant sélectionné parmi le fumarate de stéarine de sodium ou le béhénate de glycéryle
d) compresser le mélange de poudre obtenu en comprimés;
e) (éventuellement) enrober les comprimés préparés dans les étapes précédentes,
dans lequel les quantités en poids par rapport au poids de la compositions finale sont :
le sel d'hémicalcium de l'acide (E)-7-[4-(4-fluorophényl)-6-isopropyl-2-[méthyl(méthylsulfonyl)amino]pyrimidin-5-yl]-(3R, 5S)-3,5-dihydroxyhept-6-ënoïque : 5 à 20 % ;
lactose : 40 à 60 % ;
cellulose microcristalline silicifiée : 20 30 % ; et
amidon de maïs : 1 à 25 %.

5. Procédé selon la revendication 4, dans lequel le lubrifiant est le béhénate de glycéryle.

6. Compositlon pharmaceutique selon l'une quelconque des revendications 1 à 3 pour une utilisation dans le traitement de l'hypercholestérolémie, de l'hyperlipoprotéinémie et l'athérosclérose.
